(19) Europäisches Patentamt
European Patent Office
Office européen des brevets

(11) **EP 3 030 208 B1**

(12) **EUROPÄISCHE PATENTSCHRIFT**

(45) Veröffentlichungstag und Bekanntmachung des Hinweises auf die Patenterteilung:
**03.10.2018 Patentblatt 2018/40**

(21) Anmeldenummer: **15735837.5**

(22) Anmeldetag: **06.04.2015**

(51) Int Cl.:
*A61F 13/472* (2006.01)   *A61F 13/84* (2006.01)
*A61F 13/47* (2006.01)   *A61L 15/16* (2006.01)
*A61L 15/46* (2006.01)   *A61L 15/58* (2006.01)

(86) Internationale Anmeldenummer:
**PCT/DE2015/000184**

(87) Internationale Veröffentlichungsnummer:
**WO 2015/154746 (15.10.2015 Gazette 2015/41)**

(54) **VORRICHTUNG ZUR BEHANDLUNG DES UROGENITALTRAKTS VON FRAUEN**

DEVICE FOR TREATING THE FEMALE UROGENITAL TRACT

DISPOSITIF DE TRAITEMENT DE LA ZONE GÉNITO-URINAIRE DE LA FEMME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.04.2014 DE 102014005282**

(43) Veröffentlichungstag der Anmeldung:
**15.06.2016 Patentblatt 2016/24**

(73) Patentinhaber: **Tecuro AG**
**6003 Luzern (CH)**

(72) Erfinder:
• **HENGSBERGER, Corinna**
**79650 Schopfheim (DE)**
• **VON STETTEN, Otto**
**52072 Aachen (DE)**

(74) Vertreter: **Rentsch Partner AG**
**Bellerivestrasse 203**
**Postfach**
**8034 Zürich (CH)**

(56) Entgegenhaltungen:
EP-A2- 0 302 523   WO-A1-2007/073246
JP-A- 2010 259 724   US-A1- 2003 120 225

**Beschreibung**

Die Erfindung betrifft eine Vorrichtung zur Behandlung und Sanierung des Urogenitaltrakts von Frauen.

Nieren- und Harnwegsentzündungen sind eine häufige Infektionskrankheit, wobei wegen der anatomischen Verhältnisse Frauen wesentlich stärker betroffen sind als Männer. Etwa 10% der Frauen leiden an rezidivierenden Infekten mit drei und mehr Episoden im Jahr. Ursache sind hauptsächlich E. coli Bakterien, aber auch Klebsiellen, Proteus oder Staphylokokken, die aus dem eigenen Darm stammen. Sie wandern aus dem Anus über die Haut in die Urethra und schließlich in die Blase und von dort ins Nierenbecken. Selbst nach einer überstandenen Entzündung können sich am Eingang der Urethra Nischen mit überlebenden Bakterien bilden, die zu einem Rezidiv führen können.

Zur Therapie werden systemisch applizierte Antibiotika eingesetzt, die aber bei der notwendig häufigen Anwendung wegen der entstehenden Resistenzen der Bakterien unwirksam werden, sowie unerwünschte Nebenwirkungen aufweisen.

Die derzeit populäre Phytotherapie, hauptsächlich basierend auf Preiselbeeren oder Cranberry, zeigte in Studien variable Ergebnisse und konnte bisher nicht validiert werden.

In der US-Patentanmeldung US 2002/0115976 ist eine Vorrichtung zur Behandlung des Urogenitaltrakts von Frauen in Form einer Hygienebinde beschrieben, die den Anusbereich, den Damm und den Genitalbereich der Frau abdeckt. Im zentralen Dammbereich ist eine mechanische "Hürde" vorgesehen ist, die quer auf der Binde im Dammbereich zwischen Anus- und Genitalbereich verläuft. Diese "Hürde" kann anti-mikrobielle Substanzen aufweisen, sodass nach dortiger Aussage Mikro-Organismen oder Bakterien nicht vom Anus zum Genitalbereich wandern können. Mikroorganismen oder Bakterien werden aber nicht durch eine Hürde aufgehalten, selbst wenn diese anti-mikrobielle Substanzen enthält, da diese Organismen sich alle möglichen Wege suchen und durch eine mechanische Hürde nicht aufgehalten werden.

Es ist Aufgabe der Erfindung, einen neuen Therapieansatz zur Behandlung des Urogenitaltrakts von Frauen zu entwickeln und eine Vorrichtung anzugeben, mit denen zuverlässig Infektionen des Urogenitaltrakts verhindert und bestehende Infektionen wirksam behandelt werden können.

Diese Aufgabe ist durch die Merkmale des Anspruchs 1 gelöst.

In einem Aspekt umfasst die Erfindung eine Vorrichtung zur Behandlung des Urogenitaltrakts von Frauen, wobei die Vorrichtung eine Abdeckung für den urogenitalen Bereich zumindest im Bereich der Scham, der Vagina und der Urethra aufweist, und dass die Abdeckung mit einer antimikrobiell bzw. antiseptisch wirksamen Substanz gegen die Einwanderung von Bakterien in den urogenitalen Trakt präpariert ist. Die Abdeckung besteht im Wesentlichen aus drei Lagen, nämlich einer ersten Deckschicht, die mit der Haut in Kontakt kommt, einer Mittelschicht, die gegebenenfalls absorptive Eigenschaften besitzt und einer zweiten Deckschicht, die die Vorrichtung auf der anderen Seite abschliesst. Die erste Deckschicht ist mit einem antimikrobiell bzw. antiseptischen wirksamen Stoff oder Stoffgemisch präpariert. Zudem weist die Abdeckung eine längliche Form nach Art einer Hygienebinde auf, die an den Enden abgerundet ist. Des Weiteren weist eine erste Deckschicht in der Mitte in Längsrichtung eine Steppnaht auf, die das Knicken der Abdeckung erleichtert.

Gemäss der Erfindung wird statt einer systemischen Behandlung durch Medikamente, insbesondere Antibiotika, eine lokale Behandlung des Urogenitaltrakts durch antimikrobiell bzw. antiseptisch wirksame Substanzen verwendet, mit denen eine Abdeckung des urogenitalen Trakts im Wesentlichen vollständig präpariert ist. Durch diese Präparierung werden alle Bakterien vollständig eliminiert.

Die Abdeckung überdeckt vorzugsweise zumindest die Scham, die Vagina und die Urethra der Frau. Sie kann jedoch daneben auch noch den Anusbereich abdecken.n Deckschicht, die mit der Haut in Kontakt kommt, einer Mittelschicht, die gegebenenfalls absorptive Eigenschaften besitzt und einer zweiten Deckschicht, die die Vorrichtung bzw. Abdeckung auf der anderen Seite abschliesst, wobei die der Haut zugewandte Lage mit einem antimikrobiell bzw. antiseptisch wirksamen Stoff oder Stoffgemisch beschichtet ist.

Die beiden äußeren Schichten können aus demselben oder einem unterschiedlichen Material bestehen, das hautkompatibel ist. Hierfür kommen Gewebe und Non-Wovens in Frage aus z.B. Baumwolle, Polypropylen, Polyester, Polyethylen, Polyamid, Nylon, etc. Die Oberfläche der Schichten, insbesondere die der Haut zugewandte, kann gegebenenfalls strukturiert ausgebildet sein. Die Mittelschicht soll das Tragen der Vorrichtung komfortabler gestalten und kann gegebenenfalls absorptive Eigenschaften besitzen. Dazu kommen z.B. Watte, Polyurethanschaum, Polyethylen-, Polypropylengewebe oder Non-Wovens in Frage, die auch mit absorbierenden Materialien, z.B. Superabsorbern, dotiert sein können.

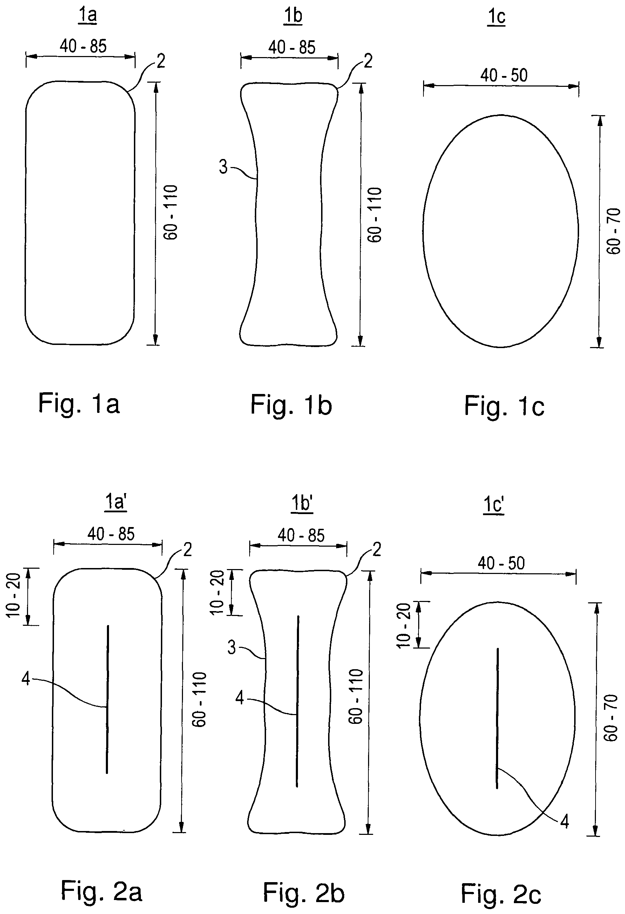

Die Form der Vorrichtung folgt den Gegebenheiten der Anatomie im Urogenitalraum. Sie ist länglich und an den Enden abgerundet, ähnlich der Form bekannter Hygienebinden. Beispiele sind in Fig. 1 gezeigt. Entscheidend für die antimikrobielle Wirksamkeit ist ein enger Kontakt der Vorrichtung mit der Haut/Schleimhaut im Urogenitalraum.

Um diesen zu verbessern, kann in einer erfindungsgemäßen Ausführungsform die der Haut zugewandte Seite der Vorrichtung eine Steppnaht aufweisen, beispielhaft dargestellt in Abb. 2, die das Knicken und so die Anpassung an die Anatomie erleichtert.

In einer weiteren Ausgestaltung der Erfindung wird dir Form der Vorrichtung so gewählt, dass diese interlabial platziert und getragen werden kann, beispielhaft in Abb. 1c und 2c dargestellt.

In einer besonders bevorzugten Ausgestaltung wird die der Haut zugewandte Seite entweder in Streifen oder punktuell mit muco- bzw. bioadhäsiven Materialien beschichtet, die die Vorrichtung auf der Haut oder Schleimhaut eng fixieren. Dafür kommen z.B. Carbomere, Poloxamere, Polyacrylate und Derivate, Chitosane und Chitosanderivate, Alginatpolyethylenglycolacrylate, Thiomere (z.B. Polycarbophil-cystein, etc.), Lectine, etc. sowie Mischungen aus den vorgenannten Materialien in Frage.

Da die Vorrichtung längere Zeit am gewählten Ort verbleibt, kann die der Haut zugewandte Schicht zusätzlich mit die Hautfeuchtigkeit erhöhenden Stoffen oder Stoffgemischen versehen werden. Hierfür sind z.B. Glyzerin, Hyaluronsäure, Glykole, Lactate, Harnstoff etc. geeignet. Dadurch wird verhindert, dass die Haut zu stark austrocknet, möglicherweise Reibungen mit der Vorrichtung entstehen, sowie die Anwendung angenehmer gestaltet.

Um die Probleme der Resistenzbildung von Bakterien zu vermeiden, werden erfindungsgemäss keine Antibiotika sondern hautverträgliche Antiseptika und antimikrobielle Substanzen verwendet, von denen keine Resistenzbildung bekannt ist. Die Substanzen werden ausgewählt aus den bekannten Wirkstoffen wie Tensiden, quaternären Ammoniumverbindungen, Biguaniden, organischen Säuren, Metallionen wie Kupfer, Zink, Silber, Gold, Ruthenium, Rhodium, Palladium, etc., Jodverbindungen, Aldehyden, etc. und Mischungen derselben. Von den vielen denkbaren Möglichkeiten sind nachfolgend drei beispielhaft aufgeführt.

In einer Ausführungsform der Erfindung wird ein Gemisch aus einem Biguanid (Polyhexamethylenbiguanid), einer quaternären Ammoniumverbindung (Benzalkoniumchlorid) und einem amphoteren Tensid (Undecylenamidopropylbetain) auf die der Haut zugewandte Seite der Vorrichtung aufgebracht.

In einer weiteren Ausführungsform wird die Vorrichtung mit einer Mischung aus Gold, Silber und Palladium beschichtet, wie sie im Handel unter dem Namen Bactiguard erhältlich ist. Eine besonders bevorzugte Ausführungsform verwendet Mischungen aus Vitamin C (Ascorbinsäure), organischen Säuren (Weinsäure, Zitronensäure), Kupfersalzen (Kupferchlorid) und einem Tensid (Laurylsulfat), wie sie in EP 2190398 beschrieben sind.

Die Vorrichtung wird mit den üblichen Verfahren zur Herstellung von Slipeinlagen, Augenkompressen und dergleichen hergestellt. Bei diesen Verfahren werden die drei Lagen der Rohlinge durch Stanzen, Verschweissen, Verkleben oder mithilfe von Mikrowellen oder Ultraschall miteinander verbunden. In einem zweiten Schritt wird die erste Deckschicht mit dem antimikrobiellen Wirkstoff bzw. dem Gemisch beschichtet. Dazu kann der Rohling in eine Wirkstofflösung getaucht, mit dieser besprüht oder bedruckt werden oder mit ähnlichen Methoden hergestellt werden.

In einem weiteren Verfahren wird die erste Deckschicht in einem ersten Schritt mit dem Wirkstoff bzw. Wirkstoffgemisch dotiert. Gegebenenfalls werden auch bio- bzw. mucoadhäsive Substanzen aufgebracht. Diese "aktive" Schicht wird in einem zweiten Schritt nach den üblichen Verfahren mit den beiden anderen Lagen verbunden.

Zur mikrobiellen Sanierung des Urogenitalraums wird die Abdeckung der erfindungsgemässen Vorrichtung in der Mitte geknickt und so platziert, dass sie die Haut und Schleimhaut des Urogenitalraums abdeckt. Auf diese Weise wird zwischen Anus und dem Urogenitalraum eine antimikrobielle Barriere aufgebaut, die die Bakterien an der Wanderung in die Urethra hindert und sie eliminiert.

In einer besonders bevorzugten Ausführungsform wird die Vorrichtung interlabial, d.h. zwischen den Schamlippen platziert, was wegen des engeren Kontakts die Effektivität noch erhöht.

Das folgende Beispiel soll die Erfindung näher erläutern, ohne ihren Umfang einzuschränken.

Eine vorgefertigte Vorrichtung, die von der Form etwa der Abbildung 1c entspricht, deren Deckschicht aus einem Mischmaterial aus Polyethylen und Polypropylen besteht, wurde mit ca. 0,4 g einer wässrigen Lösung enthaltend Laurylsulfat, Ascorbinsäure, Zitronensäure, Weinsäure, Kupferchlorid getränkt und 6 Stunden an Luft getrocknet. Die antimikrobielle Aktivität wurde in einem praxisnahen Test mit künstlicher Humanhaut und Escherichia coli (E.coli) getestet.

**Wirksamkeitstest für antibakterielle Textilien in einer Tragesimulation an menschlicher Hautflora**

Die praxisorientierte Prüfung basiert auf einer standardisierten, künstlichen/technischen Haut (HUMskin), die mit *Escherichia coli* ATCC 8739 besiedelt wird. Die HUMskin ist ein standardisiertes technisches Hautersatzmaterial auf Basis eines Biopolymers, das die Topographie und physiologischen Eigenschaften einer gesunden Haut simuliert.

Die Wirkung des Prüfmaterials kann durch eine REM-Aufnahme der HUMskin-Oberfläche mit Keimbesiedlung dargestellt werden.

Die Wirkung des Prüfmaterials gegenüber dem Testkeim wird in einer Tragesimulation auf der künstlichen Haut über einen Kontaktzeitraum von 6 Stunden (6h) quantitativ analysiert und gegen eine interne Kontrolle (Standard PES) bzw. ein Referenzmaterial bewertet.

Dazu wird die HUMskin (3 cm$^2$) zum Zeitpunkt 0 h mit einer definierten Keimzahl von *E. coli* kontaminiert (zum Zeitpunkt 0h) und mit dem Kontroll-/Referenzmaterial bzw. der Probe bedeckt und für 6 Stunden bei $36 \pm 1$ °C inkubiert (jeweils 3fach-Ansatz). Nach Ablauf der Inkubation wird die auf der HUMskin verbliebene Keimzahl bei der Kontrolle/Referenz und der Probe bestimmt (KBE absolut zum Zeitpunkt 6 h", jeweils 3fach-Ansatz).
Die antibakterielle Aktivität wird nach der Formel

$$A = (log10C6h - log10T6h)$$

mit C = Kontrollmaterial und $T$ = Probenmaterial berechnet.

Dabei wurden folgende Ergebnisse erzielt:

| Probe | KBE absolut 6Std | Log KBE | Log KBE vs. Kontrolle | Log KBE vs. Referenz |
|---|---|---|---|---|
| Kontrolle | $1,25 \times 10^6$ | 6,10 | | |
| Referenz B | $1,96 \times 10^3$ | 6,29 | -0,20 | |
| B + Wirkstoff | <20 | ≥1,28 | ≥4,82 | ≥5,01 |

Das bedeutet, dass die Bakterien zu mehr als 99,999 % reduziert wurden. Die Vorrichtung ist in hohem Maß wirksam und löst die der Erfindung zugrunde liegende Aufgabe.

In den Figuren sind schematisch Vorrichtungen bzw, Abdeckungen für den urogenitalen Trakt einer Frau dargestellt; hierbei zeigen:

**Fig. 1 a):** ein erstes Ausführungsbeispiel einer länglichen etwa reckteckigen Abdeckung;
**Fig, 1 b):** ein zweites Ausführungsbeispiel einer ebenfalls länglichen Abdeckung;
**Fig. 1c):** eine Abdeckung in ovaler Form;
**Fig, 2a), 2b) und 2c):** zu den Fig. 1a), 1b) bzw. 1 c) korrespondierende Aufsichten auf Abdeckungen gemäss der Erfindung mit jeweils einer mittleren Längsnaht.

In allen Figuren sind die Masse in Millimetern angegeben.

Eine Abdeckung 1a gemäss Fig. 1 a) ist etwa rechteckig und weist eine Länge von ca. 60 bis 110 Millimeter (mm) auf. Die Breite der Schmalseite ist 40 bis 85 mm, wobei die Abdeckung an den vier Ecken 2 abgerundet ist. Die Masse können je nach der Statur einer Frau variieren. Die Abdeckung liegt im Gebrauch über der Scham, der Vagina und der Urethra der Frau. Die Länge der Abdeckung kann grösser sein, insbesondere, wenn auch der Anus abgedeckt werden soll; sie ist dann etwa 200 mm.

Die Abdeckung 1b gemäss Fig 1 b) weist an den Längsseiten,m ausgehend von den den abgerundeten Ecken 2 longitudinale Verjüngungen 3 auf, um einen anatomisch komfortableren Sitz zu gewährleisten. Ansonsten sind die Masse ähnlich wie in Fig. 1a) .
Die Abdeckung 1c (gemäss Fig. 1c) ist oval, mit einer Länge von 60 bis 70 mm und einer Breite von 40 bis 50 mm.

Die Abdeckungen 1a', 1b' und 1c' entsprechen in den Massen jeweils den Abdeckungen 1a, 1b bzw. 1c, wobei jedoch in Längsrichtung der Abdeckung jeweils eine mittlere Naht, z.B. eine Steppnaht 4 oder dergleichen vorgesehen ist, die jeweils etwa 10 bis 20 mm vom oberen bzw. unteren Rand der Abdeckung beginnt. Hierdurch kann die Abdeckung in diesem Bereich geknickt werden, um einen engen Kontakt, auch einen interlabialen Kontakt an der Scham der Frau zu erreichen.

**Patentansprüche**

1. Vorrichtung zur Behandlung des Urogenitaltrakts von Frauen, **dadurch gekennzeichnet, dass** die Vorrichtung eine Abdeckung (1, 1a', 1 b', 1c') für den urogenitalen Bereich zumindest im Bereich der Scham, der Vagina und der Urethra aufweist, und dass die Abdeckung mit einer antimikrobiell bzw. antiseptisch wirksamen Substanz gegen die Einwanderung von Bakterien in den urogenitalen Trakt präpariert ist, **dadurch gekennzeichnet, dass** die Abdeckung aus im Wesentlichen drei Lagen besteht, nämlich einer ersten Deckschicht, die mit der Haut in Kontakt kommt, einer Mittelschicht, die gegebenenfalls absorptive Eigenschaften besitzt und einer zweiten Deckschicht, die die Vorrichtung auf der anderen Seite abschliesst, wobei die erste Deckschicht mit einem antimikrobiell bzw. antiseptischen wirksamen Stoff oder Stoffgemisch präpariert ist, und dass die Abdeckung eine längliche Form (3) nach Art einer Hygienebinde aufweist, die an den Enden (2) abgerundet ist, und dass eine erste Deckschicht in der Mitte in Längsrichtung eine Steppnaht (4) aufweist, die das Knicken der Abdeckung erleichtert.

**2.** Vorrichtung nach Anspruch 1, **dadurch gekennzeichnet, dass** die Abdeckung auch den Anusbereich der Frau abdeckt.

**3.** Vorrichtung nach einem der vorstehenden Ansprüche **dadurch gekennzeichnet, dass** die Form so gestaltet wird, dass die Vorrichtung interlabial platziert und getragen werden kann.

**4.** Vorrichtung nach einem der vorstehenden Ansprüche **dadurch gekennzeichnet, dass** die Mittelschicht aus einem Material besteht, das absorptive Eigenschaften besitzt, z.B. Watte, Polyurethanschaum, Superabsorber, etc.

**5.** Vorrichtung nach einem der vorstehenden Ansprüche **dadurch gekennzeichnet, dass** die erste und gegebenenfalls die zweite Deckschicht aus einem hautkompatiblem Gewebe besteht, z.B. Baumwolle, Polypropylen, Polyester, Polyethylen und andere non-woven Materialien.

**6.** Vorrichtung nach einem der vorstehenden Ansprüche **dadurch gekennzeichnet, dass** die erste Deckschicht entweder in Streifen oder punktuell zur besseren Fixierung auf der Haut mit bio- bzw. mucoadhäsiven Stoffen beschichtet ist.

**7.** Vorrichtung nach einem der vorstehenden Ansprüche **dadurch gekennzeichnet, dass** die erste Deckschicht zusätzlich mit die Hautfeuchtigkeit erhöhenden Stoffen beschichtet ist, z.B. Glyzerin, Hyaluronsäure , Glykolen, Harnstoff etc.

**8.** Vorrichtung nach einem der vorstehenden Ansprüche **dadurch gekennzeichnet, dass** der Wirkstoff bzw. das Wirkstoffgemisch ausgewählt wird aus den bekannten antimikrobiellen Wirkstoffen wie Tensiden, quaternären Ammoniumverbindungen, Biguaniden, organischen Säuren, Metallionen wie Kupfer, Zink, Silber, Ruthenium, Rhodium, etc., Jodverbindungen, Aldehyden, etc. und Mischungen derselben.

**9.** Vorrichtung nach einem der vorstehenden Ansprüche, **dadurch gekennzeichnet, dass** das Wirkstoffgemisch durch Tauchen, Sprühen, Drucken oder ähnliche Verfahren auf die vorgefertigte Vorrichtung aufgebracht ist.

**10.** Verfahren zur Herstellung einer Vorrichtung nach einem der Ansprüche 1 bis 9, welche mehrere Lagen aufweist, **dadurch gekennzeichnet, dass** in einem vorgelagerten Schritt die erste Deckschicht mit dem Wirkstoff bzw. Wirkstoffgemisch beschichtet und in einem zweiten Schritt mit den anderen Schichten verbunden wird.

**11.** Vorrichtung nach einem der Ansprüche 1 bis 9 zur Anwendung in der prophylaktischen Therapie, wobei die Vorrichtung interlabial platziert wird.

**Claims**

**1.** A device for treating the female urogenital tract, **characterised in that** the device comprises a covering (1, 1a', 1b', 1c') for the urogenital area at least in the area of the vulva, the vagina and the urethra, and **in that** the covering is prepared with an antimicrobially or antiseptically active substance against the migration of bacteria into the urogenital tract, **characterised in that** the covering essentially consists of three layers, namely a first cover layer which comes into contact with the skin, an intermediate layer which optionally has absorbing properties and a second cover layer which closes the device on the other side, wherein the first cover layer is prepared with an antimicrobially or antiseptically active substance or mixture of substances, and **in that** the covering has an elongated form (3) according to that of a sanitary towel which is rounded at the ends (2), and **in that** a first cover layer comprises a topstitch (4) in the middle in the longitudinal direction which simplifies the folding of the covering.

**2.** The device according to claim 1, **characterised in that** the covering also covers the female anus.

**3.** The device according to any one of the preceding claims, **characterised in that** the form is configured such that the device can be placed and worn in an interlabial manner.

**4.** The device according to any one of the preceding claims, **characterised in that** the intermediate layer consists of a material which has absorbing properties, e.g. wadding, polyurethane foam, superabsorbent polymers, etc.

**5.** The device according to any one of the preceding claims, **characterised in that** the first and optionally the second cover layer consist of a skin-compatible fabric, e.g. cotton, polypropylene, polyester, polyethylene, and other non-woven materials.

6. The device according to any one of the preceding claims, **characterised in that** the first cover layer is coated with bio- or mucoadhesive substances, either in stripes or in points, for a better fixation on the skin.

7. The device according to any one of the preceding claims, **characterised in that** the first cover layer is additionally coated with skin moisture-increasing substances, e.g. glycerol, hyaluronic acid, glycols, urea, etc.

8. The device according to any one of the preceding claims, **characterised in that** the active substance or the mixture of active substances is selected from known anti-microbial active substances, such as surfactants, quaternary ammonium compounds, biguanides, organic acids, metal ions, such as copper, zinc, silver, ruthenium, rhodium, etc., iodine compounds, aldehydes, etc. and mixtures thereof.

9. The device according to any one of the preceding claims, **characterised in that** the mixture of active substances can be applied on the pre-assembled device by immersing, spraying, pressing or any other similar method.

10. A method for manufacturing a device according to any one of claims 1 to 9 comprising several layers, **characterised in that**, in a preceding step, the first cover layer is coated with the active substance or the mixture of active substances and, in a second step, is joined with the other layers.

11. The device according to any one of claims 1 to 9 for use in prophylactic therapy, wherein the device is placed in an interlabial manner.

**Revendications**

1. Dispositif de traitement du tractus urogénital féminin, **caractérisé en ce que** le dispositif comprend un revêtement (1, 1a', 1b', 1c') pour la région urogénitale, au moins, au niveau de la vulve, du vagin et de l'urètre, et **en ce que** le revêtement est préparé avec une substance active antimicrobienne ou antiseptique contre la migration des bactéries dans le tractus urogénital, **caractérisé en ce que** le revêtement est essentiellement constitué de trois couches, en particulier, une première couche de couverture qui vient en contact avec la peau, une couche intermédiaire qui présente en option des propriétés absorbantes et une deuxième couche de couverture qui ferme le dispositif de l'autre côté, la première couche de couverture étant préparée avec une substance active ou un mélange de substances actives antimicrobiennes ou antiseptiques, et **en ce que** le revêtement a une forme allongée (3) comme une serviette hygiénique qui est arrondie aux extrémités (2), et **en ce qu'**une première couche de couverture comprend une surpiqûre (4) au milieu dans la direction longitudinale qui permet le pliage du revêtement.

2. Dispositif selon la revendication 1, **caractérisé en ce que** le revêtement recouvre également l'anus féminin.

3. Dispositif selon l'une quelconque des revendications précédentes, **caractérisé en ce que** la forme est configurée de telle sorte que le dispositif peut être placé et porté de manière interlabiale.

4. Dispositif selon l'une quelconque des revendications précédentes, **caractérisé en ce que** la couche intermédiaire est constituée d'un matériau ayant des propriétés absorbantes, p.ex. d'ouate, de mousse de polyuréthane, des superabsorbants, etc.

5. Dispositif selon l'une quelconque des revendications précédentes, **caractérisé en ce que** la première et éventuellement la deuxième couche de couverture sont constituées d'un tissu compatible avec la peau, par exemple, du coton, du polypropylène, du polyester, du polyéthylène et d'autres matériaux non tissés.

6. Dispositif selon l'une quelconque des revendications précédentes, **caractérisé en ce que** la première couche de couverture est revêtue de substances bio- ou muco-adhésives, en bandes ou en points, pour une meilleure fixation sur la peau.

7. Dispositif selon l'une quelconque des revendications précédentes, **caractérisé en ce que** la première couche de couverture est en outre revêtue de substances augmentant l'humidité de la peau, par exemple, du glycérol, de l'acide hyaluronique, de glycols, de l'urée, etc.

8. Dispositif selon l'une quelconque des revendications précédentes, **caractérisé en ce que** la substance active ou le mélange de substances actives est choisi parmi les substances actives antimicrobiennes connues, tels que les agents tensioactifs, les composés quaternaires d'ammonium, les biguanides, les acides organiques, les ions métalliques, comme le cuivre, le zinc, l'argent, le ruthénium, le rhodium, etc., les composés de l'iode, les aldéhydes, etc. et leurs mélanges.

9. Dispositif selon l'une quelconque des revendications précédentes, **caractérisé en ce que** le mélange de substances actives peut être appliqué sur le dispositif préassemblé par immersion, pulvérisation, pressage ou tout autre procédé similaire.

10. Procédé de fabrication d'un dispositif, selon l'une quelconque des revendications 1 à 9, comprenant plusieurs couches, **caractérisé en ce que**, dans une étape préliminaire, la première couche de couverture est revêtue de la substance active ou du mélange de substances actives et, dans une deuxième étape, elle est jointe avec les autres couches.

11. Dispositif selon l'une quelconque des revendications 1 à 9 pour son utilisation en thérapie prophylactique, dans lequel le dispositif est placé de manière interlabiale.

<u>1a</u>

40 - 85

2

60 - 110

Fig. 1a

<u>1b</u>

40 - 85

2

3

60 - 110

Fig. 1b

<u>1c</u>

40 - 50

60 - 70

Fig. 1c

<u>1a'</u>

40 - 85

2

10 - 20

4

60 - 110

Fig. 2a

<u>1b'</u>

40 - 85

2

10 - 20

3

4

60 - 110

Fig. 2b

<u>1c'</u>

40 - 50

10 - 20

4

60 - 70

Fig. 2c

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020115976 A
- EP 2190398 A